# EUROPEAN PATENT APPLICATION

(11) **EP 1 925 674 A1**
(43) Date of publication of application: **28.05.2008**
(21) Application number: 06124813.4
(22) Date of filing: 27.11.2006
(51) Int. Cl.: C12P 19/32, C12P 19/36, C12P 7/00, C12P 7/22, C12N 9/16

(54) **Regeneration of NADPH and/or NADH cofactors**

(71) Applicant: Universiteit van Amsterdam, 1000 GG Amsterdam (NL)
(72) Inventor: Hartog, Louis, 2015 AG Haarlem (NL); Van Herk, Teunie, 1113 HB Diemen (NL); Wever, Ron, 1622 HG Hoorn (NL)
(74) Representative: van Heuvel, Margaretha

(57) **Abstract**

The present invention discloses a method to regenerate NAD(P)H that comprises reacting together components comprising a phosphate donor, a substrate to be phosphorylated, an enzyme that catalyses both the liberation of phosphate from the phosphate donor and phosphorylation of the substrate, and an enzyme that catalyses dehydrogenation of the phosphorylated substrate and that uses NAD(P)⁺ as a cofactor such that NAD(P)H is formed.. The regeneration method may be coupled to any biosynthetic process using an enzyme requiring NAD(P)H as a cofactor.

## Description

The present invention relates to the field of biosynthesis using enzymes that require NADPH and/or NADH as cofactor, in particular relates to regeneration of these cofactors.

Several important biosynthetic processes make use of enzyme reactions that are coupled to the redox cofactor couple nicotinamide dinucleotide phosphate/reduced nicotinamide dinucleotide phosphate (NADP/NADPH), or to the redox cofactor couple nicotinamide dinucleotide/reduced nicotinamide dinucleotide (NAD/NADH). In general, in the cell the cofactors NAD/NADH are mainly related to catabolic reactions, the cofactors NADP/NADPH mainly to anabolic reactions.

Enantioselective reductions, for instance, are important reactions in the asymmetric syntheses of enantiopure compounds that are useful synthons and pharmaceutical intermediates. Enzymes can catalyze these reactions with high enantioselectivity, providing a useful and green alternative to chemical catalysis. The practical application of such bioreductions depends on the efficient and economic supply of the expensive nicotinamide cofactor NAD(P)H which is required in stoichiometric amounts.

In general, the cofactor can be recycled by the coupling of a desired enzymatic reaction with a chemical, electrochemical, or enzymatic reaction that regenerates the necessary cofactor (see for instance W.Kroutil, H.Mang, K.Edegger and K.Faber, Recent advances in the biocatalytic reduction of ketones and oxidation of sec-alcohols. Current Opinion in Chemical Biology 2004(8) 120-126). The enzymatic regeneration by "coupled substrates" and "coupled enzymes" approaches is preferred so far. Cofactor recycling has been achieved with isolated enzyme(s) and whole cells containing the necessary enzyme(s).

For instance, Wong and Whitesides (C.-H. Wong and G.M. Whitesides J.Am.Chem.Soc. 1981 103 4890-99) describe the regeneration of NADPH using glucose-6-phosphate in combination with glucose-6-phosphate dehydrogenase. Although in the method recycling of NADPH occurs effectively it has a major drawback. Glucose-6- phosphate is expensive and this hampers larges scale applications. Further glucose-6-phosphate is an effective acid catalyst for the decomposition of NAD(P)H, especially at higher concentrations.

Zang et al. (J. Zhang (2006), B. Witholt and Z.Li. Adv.Synth. Catal. 348, 429-433 describe the NADPH recycling in enantioselective bioreduction of a ketone with permeabilzed cells of a microorganism containing a keto reductase and a glucose-6-phosphate dehydrogenase. This system also requires the use of glucose-6-phosphate. The use of whole cells further depends on the amount of available intracellular cofactor which may be limiting and cannot be altered by adding extracellular cofactor unless the cell membranes are damaged in special cases during biotransformation. In addition, using whole cells has the disadvantage that the desired products may be degraded by further metabolism of the cell.

The present invention discloses a new method for regeneration of NADPH that uses cheap substrates and is very efficient.

In a first aspect, the present invention discloses a method to regenerate NAD(P)H that comprises reacting together components comprising a phosphate donor, a substrate to be phosphorylated, an enzyme that catalyses both the liberation of phosphate from the phosphate donor and phosphorylation of the substrate, and an enzyme that catalyses dehydrogenation of the phosphorylated substrate and that uses NAD(P)⁺ as a cofactor such that NAD(P)H is formed.

The terms NAD (P) and NAD(P)H as used in this invention are meant to cover NAD⁺ and NADP⁺, and NADH and NADPH, respectively.

In the method of the invention, the phosphate is efficiently recycled, allowing the method to proceed with cheap reactants like glucose and a substoichiometric amount of pyrophosphate with respect to the amount of substrate and/or product.

The phosphorylated substrate is generated using any suitable phosphate donor and a substrate that upon phosphorylation functions as a substrate for the dehydrogenation reaction.

The phosphate donor may be pyrophosphate, polyphosphate or a phosphomonoester. A suitable phosphomonoester includes a phosphorylated carbohydrate or polyalcohol, such as 6-phosphogluconate and/or glucose-6-phosphate. The liberated phosphate is transferred via a phosphate enzyme intermediate to a substrate that, when phosphorylated, functions as a substrate for an enzyme using NAD(P)⁺ as a cofactor.

The substrate to be phosphorylated is a mono- or polyhydroxy compound, preferably a carbohydrate or a polyhydric alcohol, more preferably glucose.

The enzyme that catalyzes the liberation of a phosphate from the phosphate donor advantageously also catalyzes the incorporation of this phosphate in the substrate to be phosphorylated.

Such an enzyme is a phosphatase. Phosphatases form a class of enzymes that are able to hydrolyze a wide range of phosphorylated compounds resulting in the liberation of phosphate and the corresponding alcohol. In the context of the present invention the term phosphatase is intended to mean an enzyme that is able to transphosphorylate various chemical compounds using pyrophosphate as the phosphate donor resulting in a variety of phosphorylated compounds.

To date phosphatases are widely spread in nature and have been isolated from several sources, e.g. mammals (glucose-6-phosphatase), plants, and in particular from bacterial species.

Suitable bacterial species for providing the phosphatase include *Salmonella typhimurium, Salmonella enterica Zymomonas mobilis, Morganella morganii, Shigella flexneri, Escherichia blattae, Klebsiella planticola, Prevotella intermedia.* A preferred phosphatase is obtainable from *Shigella flexneri or Salmonella enterica ser. typhimurium* (PhoNSe).

Enzymes from these bacterial species are known to be homologous to each other (Y. Mihara, T. Utagawa, H. Yamada, and Y. Asano, J. Bioscience. Bioeng., 92 (2001) 50-54) and share the three conserved sequence motifs domain 1: KXXXXXXRP, domain 2: PSGH and domain 3: SRXXXXXHXXXD, which are known and identified by J. Stuckey J and G.M. Carman, Protein Sci., 6 (1997) 469-472 and by W. Hemrika, R. Renirie, , H.L. Dekker, B. Barnett, and R. Wever, Proc. Natl. Acad. Sci. USA, 94 (1997) 2145-2149.

Phosphatases which are derived from natural phosphatases sharing these three conserved sequences and which have a better or modified enzymatic activity may also be suitable in the method of the invention. They may be obtained using mutagenesis techniques like site directed mutagenesis or directed evolution.

The phosphorylated substrate, e.g. glucose-6-phosphate, is the substrate for the dehydrogenation reaction catalyzed by a dehydrogenase. This dehydrogenase should be an enzyme using NAD(P)⁺ as a cofactor. In addition, its selection is dependent on the nature of the phosphorylated substrate and may be any dehydrogenase that is able to dehydrogenate phosphorylated carbohydrates or polyalcohols. In case of glucose-6-phosphate, the enzyme is glucose-6-phosphate dehydrogenase that may be of any source but preferably is obtainable from yeast, *Leuconostoc mesenteroides* or *Bacillus brevis.*

The dehydrogenation reaction enables regeneration of NAD(P)H. Advantageously, the dehydrogenated product resulting from the dehydrogenation reaction is in fact a phosphomonoester that can function as a phosphate donor for the phosphorylation reaction that generates the phosphorylated substrate for the dehydrogenation.

When glucose is used as the substrate to be phosphorylated, the by-product of the regeneration method is a stoichiometric amount of gluconate, which is formed from 6-P-gluconate after donating the phosphate group via a phosphorylated enzyme intermediate back to glucose.

When coupled to a reaction wherein NAD(P)H is consumed, the regeneration method of the invention enables the cycling of phosphate for at least 2 times, preferably for at least 5 times, more preferably for at least 10 times. If the reaction appears to slow down or even to cease, additional phosphate donor may be added. The addition of additional phosphate donor may continue until glucose (present in excess) is exhausted and/or any of the enzymes and/or cofactors has lost activity.

The enzymatic NAD(P)H regeneration method of the invention may be coupled to any biosynthetic process using an enzyme that requires NAD(P)H as a co-factor.

Thus, in a second aspect, the present invention discloses a method to synthesize a compound of interest from a starting compound by reacting the starting compound with an enzyme that uses NAD(P)H as a co-factor, characterized in that the NAD(P)⁺ generated during synthesis of said compound is regenerated to NAD(P)H using the method of the first aspect.

The starting compound may be a ketone, keto acid, alkane, alkene or sulfide. The choice for a particular starting compound depends on the nature of the compound of interest. The compound of interest may be a hydroxylated compound, an epoxide, a sulfoxide or an ester.

Accordingly, a biosynthetic process using the regeneration method of the invention requires the action of three enzymes, one biosynthetic enzyme using NAD(P)H as a co-factor and two enzymes for the regeneration of the consumed NAD(P)H. The additional substrates that are necessary for the regeneration according the invention advantageously comprise cheap and environmentally friendly compounds.

Such biosynthetic processes may include a bioreduction or an incorporation of an oxygen atom from dioxygen.

An example of a bioreduction process is the synthesis of enantiopure compounds, like enantiopure alcohols that serve as important intermediates for the pharmaceutical industry. Many NAD(P)H-dependent dehydrogenases are known that are useful catalysts for the synthesis of such chiral compounds from prochiral compounds. Many active and stable enzymes are available that (with high enantioselectivity) reduce ketones or keto acids to chiral alcohols, hydroxy acids or amino acids. A specific example is the production of the R or S 1-phenylethylalcohol from acetophenone using an alcohol dehydrogenase. A well known alcohol dehydrogenase is the enzyme from *Lactobaccilus kefir* (see W.Hummel Trends in Biotechnology 1999 (17) 487-492).

The incorporation of an oxygen atom from dioxygen -into hydrocarbons and other compounds is another example of one of the most useful biotransformations (see P.Meinhold, M.W.Peters, M.M.Y.Chen, K.Takahashi and F.H.Arnold. Direct conversion of ethane to ethanol by engineered cytochtome P450 BM3. Chem Bio-Chem 2005 (6) 1765-1768). Two classes of enzymes are known that catalyze these reactions, the cytochrome P450 enzymes and the flavine-containing monooxygenases. These enzymes catalyze the hydroxylation of alkanes, asymmetric epoxidation of alkenes, the sulfoxidation of sulfides and the Baeyer-Villiger-oxidations of ketones (see K.Faber, Biotransformations in Organic Chemistry 5th Edition Springer Verlag (2004) pp. 225-248).

A biosynthetic process using the regeneration method of the invention may be performed using isolated enzymes or using one or more enzymes as directly produced by a micro-organism or by permeabilized whole cells. The process may advantageously be performed in one vessel. The reaction medium and conditions may be optimized depending on the optimal reaction conditions for each enzyme. The reaction medium conveniently may be an aqueous medium or may contain organic solvents.

The biosynthetic process may be carried out at near neutral pH, typically a pH from 5-8, preferably 6-7. The substrate to be phosphorylated is present in molar excess as compared to the phosphate donor and the compound to be reduced. Typically, the substrate to be phosphorylated, e.g. glucose, is present in a concentration of 100-1000 mM, in an excess to the compound to be reduced or in which an oxygen atom is to be incorporated of 50-500 mM , in a in an excess to the initially present phosphate donor of 5-100 mM and in an excess to NAD(P)⁺ of 0.1-5 mM.

The biosynthetic product may be recovered from the reaction medium using commonly known separation and/or purification techniques. Because the process described takes place in an aqueous environment, the product is easily extracted with n-hexane or another suitable organic solvent and concentrated by evaporation.

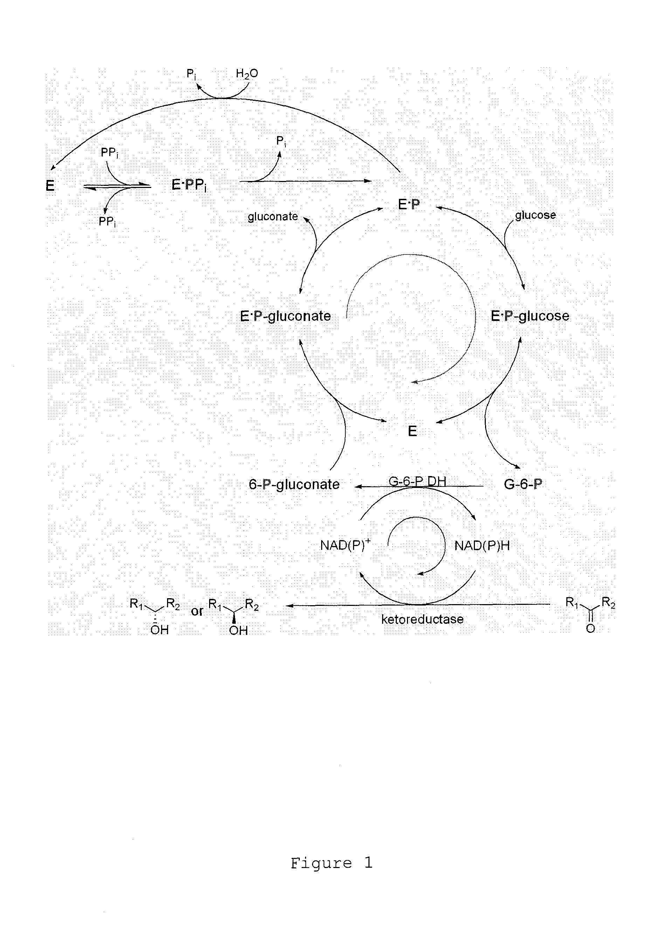

Figure 1 illustrates the process of phosphate transfer using glucose as the phosphate acceptor and pyrophosphate as the phosphate donor.
E, acid phosphatase
PPi, pyrophosphate
E.P, phosphorylating enzyme intermediate
E.P-Glucose, ternary complex between phosphorylating enzyme intermediate and glucose
E.P-Gluconate, ternary complex between phosphorylating enzyme intermediate and gluconate
G-6-PDG, glucose-6-phosphate dehydrogenase

### Examples

### Example 1: Formation of R(+)-1-phenylethylalcohol.

A reaction mixture of 1 ml was used containing 100 mM acetophenon (12 mg) in 25 mM Maleate/mM Tris buffer (pH 6.35) to which 1 mM NADP⁺, 200 mM glucose, 2 mM magnesiumchloride and 5 mM pyrophosphate were added. The reaction was initiated by the addition of 5-10 units glucose-6-phosphate dehydrogenase from yeast, 0.4 µM acid phosphatase from *Shigella flexneri* and 3 units alcohol dehydrogenase from *Lactobacillus kefir.*

This mixture was stirred at 30 °C for 24 hours in a closed vessel and extracted with 10 ml n-hexane and analyzed by HPLC using a Chiracel-OD column. The column was eluted with n-heptane-isopropanol (19:1). The HPLC effluent was monitored at 220 nm and R(+)-1-phenylethylalcohol was detected at a final concentration of 62 mM. The Borwin software program was used for HPLC data acquisition and evaluation.

Since only 5 mM pyrophosphate was used and 62 mM of alcohol was formed the phosphate group from pyrophosphate was transferred at least 12 times and apparently is transferred between glucose and gluconate by the phosphatase as illustrated in Figure 1.

### Example 2: Formation of S(-)-1-phenylethylalcohol

A reaction mixture of 1 ml was used containing 100 mM acetophenon in 100 mM phosphate buffer (pH 6.7), 0.25 mM NADP⁺, 300 mM glucose, 2 mM magnesium chloride and 5 mM pyrophosphate. To start the reaction 15 units glucose-6-phospate dehydrogenase from *Leuconostoc mesenteroides,* 0.3 µM acid phosphatase from *Shigella flexneri* and 5 units alcohol dehydrogenase from *Thermoanaerobium brockii* were added. This mixture was stirred at 30 °C for 1 day in a closed vessel.

The incubation was extracted with 10 parts n-hexane and analyzed by HPLC using a Chiralcel-OD column. The column was eluted with n-heptane-isopropanol (19:1). The HPLC effluent was monitored at 220 nm. 12 mM of S(-)-1-phenylethylalcohol was detected corresponding to a conversion of 15 %. Since 5 mM pyrophosphate was present and 12 mM of alcohol was formed the phosphate group is at least transferred 2 times by the phosphatase between glucose and gluconate.

## Claims

1. A method to regenerate NAD(P)H that comprises reacting together components comprising a phosphate donor, a substrate to be phosphorylated, an enzyme that catalyses both the liberation of phosphate from the phosphate donor and phosphorylation of the substrate, and an enzyme that catalyses dehydrogenation of the phosphorylated substrate and that uses NAD(P)⁺ as a cofactor such that NAD(P)H is formed.

2. The method according to claim 1, wherein the phosphate donor is pyrophosphate and/or a phosphomonoester, preferably is pyrophosphate, 6-phosphogluconate and/or glucose-6-phosphate.

3. The method according to claim 1, wherein the dehydrogenated phosphorylated substrate constitutes at least part of the phosphate donor.

4. The method according to claim 3, wherein the dehydrogenated phosphorylated substrate is 6-phosphogluconate.

5. The method according to claim 1, wherein the substrate to be phosphorylated is a mono- or polyhydroxy compound, preferably a carbohydrate or a polyhydric alcohol, more preferably glucose.

6. The method according to claim 1, wherein the enzyme that catalyses both the liberation of phosphate from the phosphate donor and phosphorylation of the substrate is a phosphatase, preferably obtainable from bacteria, more preferably obtainable from *Shigella flexneri* and/or *Salmonella enterica ser. typhimurium*

7. The method according to claim 1, wherein the enzyme that catalyses dehydrogenation of the phosphorylated substrate is glucose-6-phosphate dehydrogenase.

8. A method to synthesize a compound of interest from a starting compound by reacting the starting compound with an enzyme that uses NAD(P)H as a co-factor, **characterized in that** the NAD(P)⁺ generated during synthesis of said compound is regenerated to NAD(P)H using the method of any of the preceding claims.

9. The method according to claim 7, wherein the enzyme that uses NAD(P)H as a co-factor is a ketoreductase, a P450 cytochrome oxidase and/or a monooxygenase.

10. The method according to claim 7 wherein the compound of interest is a hydroxylated compound, preferably a chiral alcohol, an epoxide, a sulfoxide or an ester.
